# EUROPEAN PATENT SPECIFICATION

(11) **EP 3 843 723 B1**
(45) Date of publication and mention of the grant of the patent: **07.01.2026**
(21) Application number: 19855537.7
(22) Date of filing: 28.08.2019
(51) Int. Cl.: A61K 31/4184, A61K 31/43, A61K 31/7048, A61K 9/00, A61P 31/04, A61K 9/20, A61P 1/04

(54) **COMPOSITION FOR ERADICATING HELICOBACTER PYLORI**
ZUSAMMENSETZUNG ZUR ERADIKATION VON HELICOBACTER PYLORI
COMPOSITION DESTINÉE À ÉRADIQUER HELICOBACTER PYLORI

(30) Priority: 29.08.2018 KR 20180102250
(43) Date of publication of application: 07.07.2021
(73) Proprietor: HK inno.N Corporation, Seoul 04551 (KR)
(72) Inventor: KIM, Bong Tae, Hanam-si Gyeonggi-do 12904 (KR); KIM, Dongkyu, Suwon-si Gyeonggi-do 16694 (KR); KIM, Eun Ji, Seoul 06751 (KR); LEE, Ji Won, Seongnam-si Gyeonggi-do 13162 (KR); OH, Kyeongmin, Seoul 03304 (KR); KIM, Ahrong, Seoul 08845 (KR); SONG, Geun Seog, Seoul 02834 (KR); RYU, Shin-Young, Namyangju-si Gyeonggi-do 12273 (KR); KIM, Eun Kyung, Seoul 05112 (KR); SHIN, Naree, Incheon 21348 (KR); KANG, Hyun Ji, Seoul 07691 (KR); KIM, Jae Min, Seoul 04559 (KR); PARK, Yu-Gyeong, Seoul 04559 (KR); JEONG, Haneul, Seoul 07229 (KR)
(74) Representative: Kraus & Lederer PartGmbB
(86) International application number: PCT/KR2019/011017
(87) International publication number: WO 2020/045992

(56) References cited:
- WO-A1-2017/125912
- CN-A- 101 015 694
- CN-A- 101 015 694
- CN-A- 108 379 258
- US-A1- 2003 036 533
- US-A1- 2007 142 448
- US-A1- 2007 142 448
- JONG-LYUL GHIM ET AL: "Pharmacokinetics and Pharmacodynamics of Tegoprazan Coadministered With Amoxicillin and Clarithromycin in Healthy Subjects", THE JOURNAL OF CLINICAL PHARMACOLOGY, LIPPINCOTT CO, HAGERSTOWN, MD, US, vol. 61, no. 7, 12 January 2021 (2021-01-12), pages 913 - 922, XP071411290, ISSN: 0091-2700, DOI: 10.1002/JCPH.1805

## Description

### [Technical Field]

The present invention relates to a composition for use in eradicating *Helicobacter pylori* and a use thereof.

### [Background Art]

*Helicobactor pylori* (H.P bacterium, *H. pylori*)*,* which is a bacterium involved in the occurrence of various gastrointestinal disorders such as gastritis, ulcers, etc., does serious damage to human health.

*Helicobacter pylori* reproduces in the gastric mucous membrane of humans. It is known that *Helicobacter pylori* is a causative pathogen of gastritis, gastric ulcers and duodenal ulcers, and is also connected with diseases such as gastric mucosa-associated lymphoid tissue (MALT) lymphomas, atrophic gastritis, gastric hyperplastic polyps, etc. Once *Helicobacter pylori* settles in the gastric mucous membrane, such bacterium is not removed out, but continues to reproduce in the stomach in spite of strong immune responses to infections. Also, pH is kept very low in the stomach due to hydrochloric acid, and thus many antibiotics become inactive.

Now, it is known that a triple combination therapy with antibiotics (amoxicillin and clarithromycin) and a proton pump inhibitor is partially used to eradicate *Helicobacter pylori.* However, with an advent of bacteria resistant to the antibiotics, it has become difficult to carry out complete eradication with such method.

For example, a rate of success in eradicating *Helicobacter pylori* has been recently decreased to 80% or lower worldwide, and to even 70% or lower in some countries. According to the studies conducted in South Korea for the recent five years from 2011 to 2015, it was also found that an eradication rate of *Helicobacter pylori* is decreased to 70.7% (58.7-80.0%) in an intention-to-treat (ITT) analysis and to 76.2% (64.5-87.5%) in a per-protocol (PP) analysis. One of the factors of such decrease in the eradication rate is an increase in resistance of *Helicobacter pylori* to the antibiotics, which are used as a therapeutic medicine. In particular, its growing resistance to clarithromycin becomes a key factor of failed treatment in a standard triple therapy. Thus, second-, third-line eradication therapies, etc., based on new antibiotics (metronidazole and levofloxacin) have been used but with much limitation, too because such eradication methods also have an influence on an action of beneficial enterobacteria in the human body, thus causing a problem of side effects such as diarrhea, etc.

As seen above, in case of administering the antibiotics for a long period of time, it is feared that there occurs a very serious problem about a growth in resistant bacteria. In particular, a frequency of bacteria resistant to clarithromycin tends to show a rapid increase. In an example of infections with resistant bacteria, it has been reported that an eradication rate is remarkably decreased and there occurs acquisition of resistance to clarithromycin after failed eradication. In other words, it is thought that an insufficient eradication treatment performed carelessly has increased an advent of resistant bacteria. Thus, it is very important to provide an optimal environment sufficient for the eradication treatment.

Another important factor in the eradication treatment is to maintain an intragastric pH to be at least 5 or more such that an action of the antibiotics may be optimized. It has been reported that the intragastric pH is 5.0-7.6 for 24 hours in those patients successful in the eradication treatment, while such pH is 2.2-6.2 in failure cases. Thus, such report indicates that a highly maintained pH in the stomach is an important condition for achieving the successful eradication treatment.

In other words, there is a need for developing and using a drug, which may inhibit gastric acid from being secreted for an appropriate period of time to raise pH close to a pKa value unique to antibiotics, thereby increasing stability of the antibiotics and reducing a minimum inhibitory concentration of the antibiotics on *Helicobacter pylori.*

### [Disclosure]

### [Technical Problem]

Against these backdrops, the present inventors have made every endeavor to develop a suitable composition for eradicating *Helicobacter pylori,* and thus have identified that a certain benzimidazole derivative compound shows an excellent effect on a pH growth in the stomach and exhibits a remarkable effect on eradicating *Helicobacter pylori* by being used together with amoxicillin and clarithromycin, thereby completing the present invention.

The present invention relates to the subject-matter of the claims. The present invention provides a pharmaceutical composition for use in eradicating *Helicobacter pylori,* containing: a compound represented by a following formula 1, optical isomers thereof or pharmaceutically acceptable salts thereof; amoxicillin or pharmaceutically acceptable salts thereof; and clarithromycin or pharmaceutically acceptable salts thereof as an active ingredient:

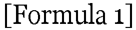

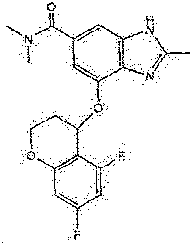

The present invention provides a combination, containing: a compound represented by the formula 1 above, optical isomers thereof or pharmaceutically acceptable salts thereof; amoxicillin or pharmaceutically acceptable salts thereof; and clarithromycin or pharmaceutically acceptable salts thereof.

The present invention provides a kit including a combination for use in eradicating *Helicobacter pylori,* containing: a compound represented by the formula 1 above, optical isomers thereof or pharmaceutically acceptable salts thereof; amoxicillin or pharmaceutically acceptable salts thereof; and clarithromycin or pharmaceutically acceptable salts thereof.

Disclosed herein is a method for eradicating *Helicobacter pylori,* including a step of administering a pharmaceutical composition, containing: a compound represented by the formula 1 above, optical isomers thereof or pharmaceutically acceptable salts thereof; amoxicillin or pharmaceutically acceptable salts thereof; and clarithromycin or pharmaceutically acceptable salts thereof as an active ingredient, into subjects in need.

Disclosed herein is a use of a compound represented by the formula 1 above, optical isomers thereof or pharmaceutically acceptable salts thereof; amoxicillin or pharmaceutically acceptable salts thereof; and clarithromycin or pharmaceutically acceptable salts thereof, in preparing a drug for eradicating *Helicobacter pylori.*

Disclosed herein is a use of a compound represented by the formula 1 above, optical isomers thereof or pharmaceutically acceptable salts thereof; amoxicillin or pharmaceutically acceptable salts thereof; and clarithromycin or pharmaceutically acceptable salts thereof, in eradicating *Helicobacter pylori.*

### [Technical Solution]

The present invention provides a pharmaceutical composition for use in eradicating *Helicobacter pylori,* containing: a compound represented by a following formula 1, optical isomers thereof or pharmaceutically acceptable salts thereof; amoxicillin or pharmaceutically acceptable salts thereof; and clarithromycin or pharmaceutically acceptable salts thereof as an active ingredient:

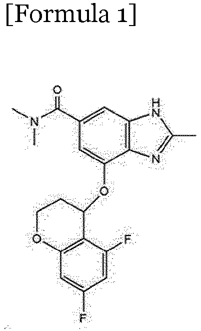

The compound represented by the formula 1 above is also named "4-(5,7-difluorochroman-4-yloxy)-N,N,2-trimethyl-1H-benzo[d]imidazole-6-carboxamide."

The compound may be isolated from a natural source of supply; may be prepared with chemical modification after being obtained from the natural source of supply; or may be prepared by those skilled in the art with chemical synthesis according to a known synthesis method (International Patent Publication WO2007/072146). Also, commercially manufactured goods may be purchased and used as the compound.

In case of the compound represented by the formula 1 above in the present invention, the compound or pharmaceutically acceptable salts thereof as well as solvates, hydrates and stereoisomers, which may be prepared therefrom and have the same efficacy thereas, are all included within the scope of the present invention.

Particularly, the compound represented by the formula 1 above may be a compound represented by a following formula 2:

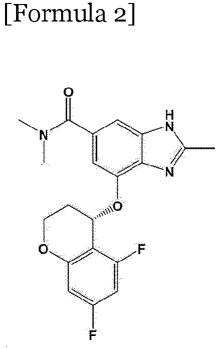

The pharmaceutical composition of the present invention may contain the compound represented by the formula 1 above, optical isomers thereof or pharmaceutically acceptable salts thereof in an amount of 10 to 500 mg, particularly in an amount of 30 to 200 mg, and more particularly in an amount of 100 to 200 mg, but not limited thereto.

Amoxicillin is named "(2S,5R,6R)-6-{[(2R)-2-amino-2-(4-hydroxyphenyl) acetyl]amino}-3,3-dimethyl-7-oxo-4-thia-1-azabicyclo[3.2.0]heptane-2-carboxylic acid" and has a structure of a following formula 3:

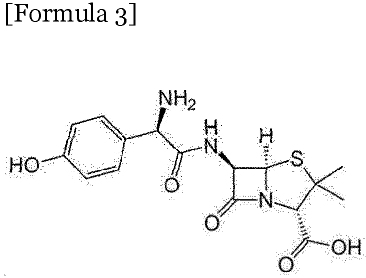

The compound may be isolated from a natural source of supply; may be prepared with chemical modification after being obtained from the natural source of supply; or may be prepared by those skilled in the art with chemical synthesis according to a known synthesis method. Also, commercially manufactured goods may be purchased and used as the compound.

In case of the compound represented by the formula 3 above in the present invention, the compound or pharmaceutically acceptable salts thereof as well as solvates and hydrates, which may be prepared therefrom and have the same efficacy thereas, are all included within the scope of the present invention.

The pharmaceutical composition of the present invention for use in eradicating *Helicobacter pylori* may contain amoxicillin or pharmaceutically acceptable salts thereof in an amount of 100 mg to 4 g, particularly in an amount of 500 mg to 2.5 g, and more particularly in an amount of 1 g to 2 g, but not limited thereto.

Clarithromycin is named "(3R,4S,5S,6R,7R,9R,11R,12R,13S,14R)-6-[(2S,3R,4S,6R)-4-(dimethylamino)-3-hydroxy-6-methyloxane-2-yl]oxy-14-ethyl-12,13-dihydroxy-4-[(2R,4R,5S,6S)-5-hydroxy-4-methoxy-4,6-dimethyloxane-2-yl]oxy-7-methoxy-3,5,7,9,11,13-hexamethyl-oxacyclotetradecane-2,10-dione" and has a structure of a following formula 4:

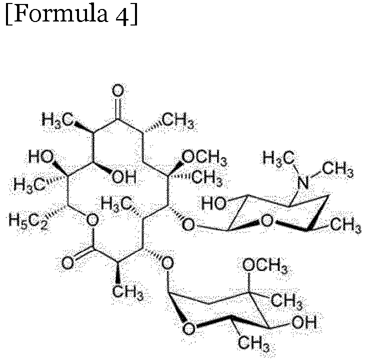

The compound may be isolated from a natural source of supply; may be prepared with chemical modification after being obtained from the natural source of supply; or may be prepared by those skilled in the art with chemical synthesis according to a known synthesis method. Also, commercially manufactured goods may be purchased and used as the compound.

In case of the compound represented by the formula 4 above in the present invention, the compound or pharmaceutically acceptable salts thereof as well as solvates and hydrates, which may be prepared therefrom and have the same efficacy thereas, are all included within the scope of the present invention.

The pharmaceutical composition of the present invention for use in eradicating *Helicobacter pylori* may contain clarithromycin or pharmaceutically acceptable salts thereof in an amount of 50 mg to 3 g, particularly in an amount of 100 mg to 1g, and more particularly in an amount of 500 mg to 1 g, but not limited thereto. In the present invention, "pharmaceutically acceptable salts" mean the salts formed with any inorganic acid, organic acid or base, which neither causes a serious stimulus to organic bodies dosed therewith, nor does damage to biological activity and physical property of the compound. As salts, the followings may be used: the salts conventionally used in the art, such as acid-addition salts formed with pharmaceutically acceptable free acid.

*"Helicobacter pylori"* is a bacterium, which is identified as a causative pathogen of chronic gastritis, gastric/duodenal ulcers, stomach cancer, etc., while proliferating in the gastric mucous membrane of the human body. As a gram-negative bacillus with several flagellums, this bacterium proliferates in a surface or mucus of a gastric mucous membrane layer. *Helicobacter pylori,* which is a spiral-shaped gram-negative bacillus with motility, lives in a mucus layer of the gastric mucous membrane and has an enzyme called urease to decompose urea and produce ammonia therefrom, such that *Helicobacter pylori* uses such ammonia to neutralize a strong acid environment in the stomach and survives therein.

It has been recently known that *"Helicobacter pylori"* has resistance to various antibiotics. For example, resistant strains may be defined by means of minimum inhibitory concentration (MIC) values. The MIC means a minimum concentration, which is required for drugs such as antibiotics, etc., to inhibit a growth of bacteria.

Particularly, those strains having an MIC value of 0.03 µg/ml or more, preferably 0.5 µg/ml or more with regard to amoxicillin, may be defined as the strains resistant to amoxicillin.

Particularly, those strains having an MIC value of 1 µg/ml or more, preferably 1.5 µg/ml or more with regard to clarithromycin, may be defined as the strains resistant to clarithromycin.

*Helicobacter pylori* resistant to antibiotics means *Helicobacter pylori,* which shows resistance to the antibiotics due to a continuous use of antibiotic drugs or a variation of *Helicobacter pylori* itself.

In the present invention, "eradication" includes removing microorganisms from a place where they are present, or inhibiting the proliferation and growth thereof all. In the present invention, the eradication of *Helicobacter pylori* includes removing *Helicobacter pylori* present in the stomach, or inhibiting the proliferation and growth thereof all.

The composition of the present invention shows an excellent effect of eradicating even *Helicobacter pylori* resistant to antibiotics, and thus is effectively used in eradicating resistant strains.

In the present invention, "prevention" includes all the acts of inhibiting or delaying the growth of *Helicobacter pylori* in advance with administration of the composition of the present invention, while "treatment" includes all the acts of eradicating *Helicobacter pylori* with the composition of the present invention, thus improving or beneficially changing diseases such as chronic gastritis, gastric/duodenal ulcers, stomach cancer, etc., which occur due to *Helicobacter pylori* as a causative pathogen.

The compound represented by the formula 1 of the present invention acts as a potassium-competitive acid blocker (P-CAB) to maintain an intragastric pH at 5 or more, particularly at 5.5 or more, and more particularly at 6.0 or more, such that the composition maintains pH close to a pKa value of antibiotics, i.e., amoxicillin and clarithromycin, and thus enhances stability of the antibiotics and greatly reduces a minimum inhibitory concentration of the antibiotics. Also, such compound has a long half life to increase the intragastric pH for a certain period of time or longer, such that the compound maximizes an eradication effect of amoxicillin and clarithromycin.

When the compound of Formula 1 of the present invention was administered with amoxicillin and clarithromycin in combination, Cmax (maximum serum concentration) and AUC (area under the curve) of the compound of formula 1 and clarithromycin increased, and it was confirmed that the bioavailability of drugs had increased after administering three formulations in combination.

Also, such compound shows an excellent effect of eradicating even *Helicobacter pylori* resistant to antibiotics, and thus may be effectively used in eradicating resistant strains. Furthermore, such compound continues to have an effect of maintaining pH at a certain level or more for a long time, and thus has an advantage of achieving a remarkable compliance with medication.

Particularly, in case of the compound represented by the formula 1 of the present invention, a percentage of time at which an intragastric pH is maintained at more than 5, particularly a percentage of time at which an intragastric pH is maintained at more than 5.5, and more particularly more than 6.0 for a period of up to 24 hours after administration thereof, is at least 60%, at least 70%, at least 80% and at least 90%, such that the compound maintains a pH value at a high level for a long period of time and thus maximizes an eradication effect of amoxicillin and clarithromycin.

Also, the compound represented by the formula 1 of the present invention rapidly increases the intragastric pH to at least 5, at least 5.5 and at least 6.0 within 3 hours, particularly within 2.5 hours, and more particularly within 2 hours after administration thereof, and thus maximizes an eradication effect of amoxicillin and clarithromycin.

A pharmaceutical composition for use in eradicating *Helicobacter pylori,* containing: the compound represented by the formula 1 of the present invention, optical isomers thereof or pharmaceutically acceptable salts thereof; amoxicillin or pharmaceutically acceptable salts thereof; and clarithromycin or pharmaceutically acceptable salts thereof may further contain suitable carriers, excipients or diluents, which are conventionally used.

The compound represented by the formula 1, optical isomers thereof or pharmaceutically acceptable salts thereof; amoxicillin or pharmaceutically acceptable salts thereof; and clarithromycin or pharmaceutically acceptable salts thereof may further contain suitable carriers, excipients or diluents, which are conventionally used by each thereof, and thus may be formulated into dosage forms.

The compound represented by the formula 1, optical isomers thereof or pharmaceutically acceptable salts thereof; amoxicillin or pharmaceutically acceptable salts thereof; and clarithromycin or pharmaceutically acceptable salts thereof may further contain suitable carriers, excipients or diluents, which are conventionally used by at least two thereof, and thus may be formulated into dosage forms.

The compound represented by the formula 1, optical isomers thereof or pharmaceutically acceptable salts thereof; amoxicillin or pharmaceutically acceptable salts thereof; and clarithromycin or pharmaceutically acceptable salts thereof may further contain suitable carriers, excipients or diluents, which are conventionally used by all thereof together, and thus may be formulated into one dosage form.

In the present invention, "pharmaceutically acceptable carriers" include carriers or diluents, which neither irritate organisms nor inhibit the biological activity and characteristics of an injected compound. Types of the carriers usable in the present invention are not particularly limited, and any carriers may be used, as long as they are conventionally used in the art and pharmaceutically acceptable.

A non-limiting example of the carriers may include saline solution, sterilized water, Ringer's solution, buffered saline, albumin injection solution, dextrose solution, maltodextrin solution, glycerol, ethanol, starch, lactose, mannitol, etc. Such carriers may be used alone, or may be used in such a way that at least two thereof are mixed together. Also, such carriers may be used with the addition of other conventional additives, such as antioxidants and/or buffer solutions, etc., if necessary.

If the compound represented by the formula 1 of the present invention, optical isomers thereof or pharmaceutically acceptable salts thereof are formulated into a preparation, the carriers may be contained in an amount of 0.01 to 50.0 wt%, and particularly in an amount of 0.1 to 10 wt% with regard to the total weight of the preparation, but not limited thereto.

If amoxicillin or pharmaceutically acceptable salts thereof are formulated into a preparation, the carriers may be contained in an amount of 0.01 to 50.0 wt%, and particularly in an amount of 0.1 to 10 wt% with regard to the total weight of the preparation, but not limited thereto.

If clarithromycin or pharmaceutically acceptable salts thereof are formulated into a preparation, the carriers may be contained in an amount of 0.01 to 50.0 wt%, and particularly in an amount of 0.1 to 10 wt% with regard to the total weight of the preparation, but not limited thereto.

In the present invention, "administration" means introducing the pharmaceutical composition of the present invention into subjects in question by means of any appropriate method, and such administration may be performed via various oral or parenteral routes, as long as such composition may reach a target tissue. Particularly, such composition may be administered via an oral administration mode, but not limited thereto. The compound represented by the formula 1, optical isomers thereof or pharmaceutically acceptable salts thereof; amoxicillin or pharmaceutically acceptable salts thereof; or clarithromycin or pharmaceutically acceptable salts thereof may be respectively formulated into a separate dosage form to be administered, and may be formulated into one dosage form to be administered.

In the present invention, a frequency of administering the compound represented by the formula 1, optical isomers thereof or pharmaceutically acceptable salts thereof; amoxicillin or pharmaceutically acceptable salts thereof; and clarithromycin or pharmaceutically acceptable salts thereof may vary depending on various factors, including an age, weight, gender, severity and administered dose. For example, such administration may be performed once a day, twice a day, three times a day, once per two days, once per three days, once per four days, once per five days, once per six days or once a week. Particularly, such administration is performed once a day or twice a day, and maintains an intragastric pH at a certain level or more for a certain period of time or longer within such frequency of administration, thereby maintaining pH close to a pKa value of amoxicillin and clarithromycin to enhance stability of the antibiotics, and thereby greatly reducing a minimum inhibitory concentration of the antibiotics to maximize an eradication effect of amoxicillin and clarithromycin.

In the present invention, the pharmaceutical composition for use in eradicating *Helicobacter pylori* may be administered into subjects infected with *Helicobacter pylori,* wherein the *Helicobacter pylori* may be resistant to antibiotics, and particularly may be resistant to amoxicillin or clarithromycin, but not limited thereto.

Disclosed herein is a method for eradicating *Helicobacter pylori,* including a step of administering the pharmaceutical composition into subjects in need.

The pharmaceutical composition may be administered in a pharmaceutically effective amount to eradicate *Helicobacter pylori.* The pharmaceutical composition may be administered alone or used in combination with a surgery, endocrinotherapy, chemotherapy and methods of using a biologic response modifier.

In the present invention, any subjects may be applicable without a particular limitation, as long as they need an eradication of *Helicobacter pylori,* and in particular are suspected of being infected with *Helicobacter pylori,* as a cause of chronic gastritis, gastric/duodenal ulcers, stomach cancer, etc. Particularly, such subjects include all the animals, including humans and non-humans such as monkeys, dogs, cats, rabbits, guinea pigs, rats, mice, cows, sheep, pigs, goats, etc., and may be efficiently treated by administering the pharmaceutical composition containing the inventive compound or pharmaceutically acceptable salts thereof into subjects in question.

Disclosed herein is a use of the compound represented by the formula 1 above, optical isomers thereof or pharmaceutically acceptable salts thereof; amoxicillin or pharmaceutically acceptable salts thereof; and clarithromycin or pharmaceutically acceptable salts thereof, in preparing a drug for eradicating *Helicobacter pylori.* To prepare a drug for eradicating *Helicobacter pylori,* the compound represented by the formula 1 above or pharmaceutically acceptable salts thereof; amoxicillin or pharmaceutically acceptable salts thereof; and clarithromycin or pharmaceutically acceptable salts thereof may be mixed with acceptable adjuvants, diluents, carriers, etc., and may be prepared into complex preparations along with other active agents, thus having a synergy action of active components.

Disclosed herein is a use of the compound represented by the formula 1 above, optical isomers thereof or pharmaceutically acceptable salts thereof; amoxicillin or pharmaceutically acceptable salts thereof; and clarithromycin or pharmaceutically acceptable salts thereof, in eradicating *Helicobacter pylori.* The compound represented by the formula 1 above, optical isomers thereof or pharmaceutically acceptable salts thereof; amoxicillin or pharmaceutically acceptable salts thereof; and clarithromycin or pharmaceutically acceptable salts thereof may be mixed with acceptable adjuvants, diluents, carriers, etc., and may be prepared into complex preparations along with other active agents, thus having a synergy action of active components.

The present invention provides a composition for use in eradicating *Helicobacter pylori,* containing: the compound represented by the formula 1 above, optical isomers thereof or pharmaceutically acceptable salts thereof; amoxicillin or pharmaceutically acceptable salts thereof; and clarithromycin or pharmaceutically acceptable salts thereof.

The present invention provides a combination, containing: the compound represented by the formula 1 above, optical isomers thereof or pharmaceutically acceptable salts thereof; amoxicillin or pharmaceutically acceptable salts thereof; and clarithromycin or pharmaceutically acceptable salts thereof.

Particularly, the present invention provides a combination for eradicating *Helicobacter pylori,* containing: the compound represented by the formula 1 above, optical isomers thereof or pharmaceutically acceptable salts thereof; amoxicillin or pharmaceutically acceptable salts thereof; and clarithromycin or pharmaceutically acceptable salts thereof.

In the present invention, the combination refers to any unions between at least two preparations. The combination may be at least two separate preparations, and may be mixtures thereof or any modifications thereof. In other words, the combination may contain each of separate preparations, and may be formed with one preparation.

Such combination may be formed into a kit type. The kit includes each of separate preparations, and may optionally include other elements, for example, additional reagents, user's manuals or the like.

In other words, the present invention provides the kit including the combination for use in eradicating *Helicobacter pylori,* containing: the compound represented by the formula 1 above, optical isomers thereof or pharmaceutically acceptable salts thereof; amoxicillin or pharmaceutically acceptable salts thereof; and clarithromycin or pharmaceutically acceptable salts thereof.

Matters mentioned in the composition of the present invention are also equally applied to the combination, kit, therapeutic method and use, if not contradictory to each other.

### [Advantageous Effects]

A pharmaceutical composition of the present invention for use in eradicating *Helicobacter pylori* maintains an intragastric pH at a certain level or more for a certain period of time or longer, thereby maintaining pH close to a pKa value of amoxicillin and clarithromycin to enhance stability of antibiotics, and thereby greatly reducing a minimum inhibitory concentration of the antibiotics to maximize an eradication effect of amoxicillin and clarithromycin. Also, such composition shows an excellent effect of eradicating even *Helicobacter pylori* resistant to the antibiotics, and thus may be also effectively used in eradicating resistant strains, and such composition continues to have an effect of maintaining pH at a certain level or more for a long time, and thus has an advantage of a remarkable compliance with medication, too. Furthermore, such composition has an advantage, in that it may be available before or after meals without considering a diet therapy, unlike existing combination therapies.

### [Description of Drawings]

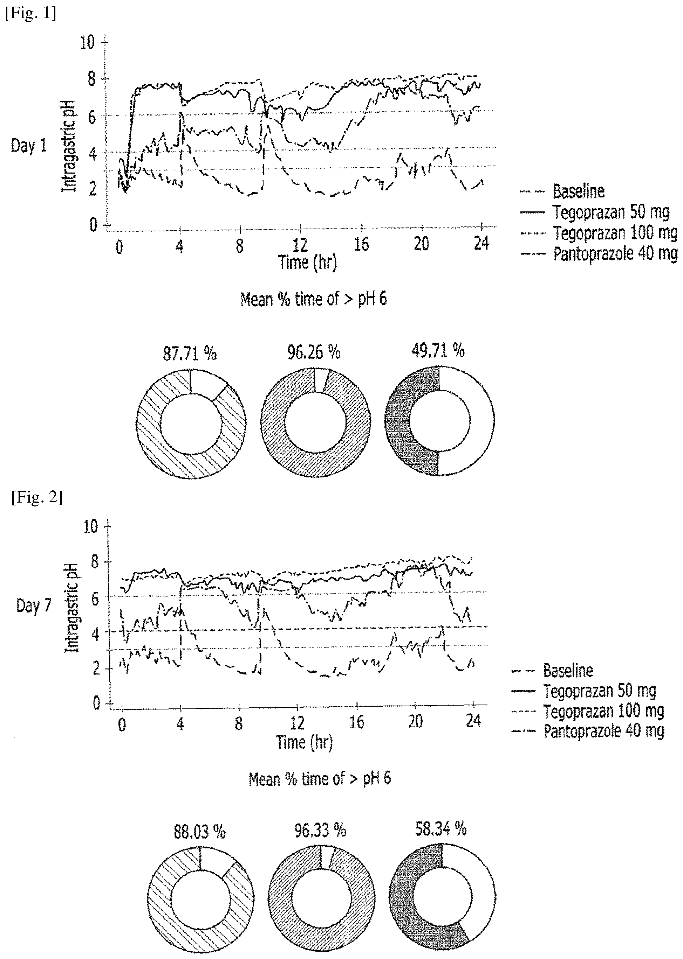

Fig. 1 is a graph of showing a change of an intragastric pH and a percentage of time at pH 6 or more in individuals on Day 1 after administering the inventive composition; and pantoprazole, amoxicillin and clarithromycin as a control group into the individuals (a left circle refers to Tegoprazan 50 mg; a middle circle does Tegoprazan 100 mg; and a right circle does Pantoprazole 40 mg).
Fig. 2 is a graph of showing a change of an intragastric pH and a percentage of time at pH 6 or more in individuals on Day 7 after administering the inventive composition; and pantoprazole, amoxicillin and clarithromycin as a control group into the individuals (a left circle refers to Tegoprazan 50 mg; a middle circle does Tegoprazan 100 mg; and a right circle does Pantoprazole 40 mg).

### [Mode for Invention]

Hereinafter, the present invention will be described in more detail through exemplary embodiments. These exemplary embodiments are provided only for the purpose of illustrating the present invention in more detail, and thus the scope of the present invention is not limited thereto.

### Example 1: CJ-12420 (Tegoprazan) 50 mg, amoxicillin and clarithromycin

### (1) Tablet containing 50 mg of CJ-12420 (Tegoprazan)

A preparation was produced according to a following procedure, in order to prepare 50 mg of CJ-12420. A dosage form was prepared to contain 50 mg of 4-[(5,7-difluoro-3,4-dihydro-2H-chromene-4-yl)oxy]-N,N,2-trimethyl-1H-benzimidazole-6-carboxamide as a main component. Mannitol, microcrystalline cellulose and croscarmellose sodium were mixed in the main component, wherein fillers were contained therein at a rate of 1 to 99% (50 mg of mannitol and 80 mg of microcrystalline cellulose) with regard to parts by weight of a final dosage form, and a rate of disintegrants was used within a range of 1 to 20% (10 mg of croscarmellose sodium) with regard to parts by weight of the final dosage form to prepare a mixture.

A binder solution containing hydroxypropyl cellulose and purified water was added to the resulting mixture, and subjected to granulation, wherein a content of binders was used within a range of 4 to 40% (6 mg of hydroxypropyl cellulose) with regard to parts by weight of an active component to prepare granules.

A process of drying the granules was performed, after which milling was carried out, such that resulting size-regulated products, microcrystalline cellulose, croscarmellose sodium, colloidal silicon dioxide and magnesium stearate were added to the granules and mixed together.

A rate of diluents was used within a range of 1 to 10% (2 mg of colloidal silicon dioxide) with regard to parts by weight of the final dosage form, and a rate of glydents was used within a range of 1 to 10% (2 mg of magnesium stearate) with regard to parts by weight of the final dosage form, after which a resulting mixture was compressed and prepared into a tablet.

The tablet was coated with a film coating agent. The tablet was prepared in such a way that coating was formed at a weight ratio of 2 to 6% (6 mg) with regard to parts by weight of the final dosage form.

### (2) Amoxicillin or clarithromycin tablet

1000 mg of Kymoxin^{®} capsule of Yuhan Corp., was used for amoxicillin, while 500 mg of Klaricid^{®} film coated tablet of Abbott Korea Co., Ltd., was used for clarithromycin.

### Example 2: CJ-12420 (Tegoprazan) 100 mg, amoxicillin and clarithromycin

### (1) Tablet containing 100 mg of CJ-12420 (Tegoprazan)

A preparation was produced according to a following procedure, in order to prepare 100 mg of CJ-12420. A dosage form was prepared to contain 100 mg of 4-[(5,7-difluoro-3,4-dihydro-2H-chromene-4-yl)oxy]-N,N,2-trimethyl-1H-benzimidazole-6-carboxamide as a main component. Mannitol, microcrystalline cellulose and croscarmellose sodium were mixed in the main component, wherein fillers were contained therein at a rate of 1 to 99% (100 mg of mannitol and 160 mg of microcrystalline cellulose) with regard to parts by weight of a final dosage form, and a rate of disintegrants was used within a range of 1 to 20% (20 mg of croscarmellose sodium) with regard to parts by weight of the final dosage form to prepare a mixture.

A binder solution containing hydroxypropyl cellulose and purified water was added to the resulting mixture, and subjected to granulation, wherein a content of binders was used within a range of 4 to 40% (12 mg of hydroxypropyl cellulose) with regard to parts by weight of an active component to prepare granules.

A process of drying the granules was performed, after which milling was carried out, such that resulting size-regulated products, microcrystalline cellulose, croscarmellose sodium, colloidal silicon dioxide and magnesium stearate were added to the granules and mixed together.

A rate of diluents was used within a range of 1 to 10% (4 mg of colloidal silicon dioxide) with regard to parts by weight of a final dosage form, and a rate of glydents was used within a range of 1 to 10% (4 mg of magnesium stearate) with regard to parts by weight of the final dosage form, after which a resulting mixture was compressed and prepared into a tablet.

The tablet was coated with a film coating agent. The tablet was prepared in such a way that coating was formed at a weight ratio of 2 to 6% (12 mg) with regard to parts by weight of the final dosage form.

### (2) Amoxicillin or clarithromycin tablet

1000 mg of Kymoxin^{®} capsule of Yuhan Corp., was used for amoxicillin, while 500 mg of Klaricid^{®} film coated tablet of Abbott Korea Co., Ltd., was used for clarithromycin.

### Comparative Example 1: Pantoprazole, amoxicillin and clarithromycin

40 mg of Pantoloc^{®} tablet of Takeda Pharmaceutical Co. Ltd., was used for pantoprazole; 1000 mg of Kymoxin^{®} capsule of Yuhan Corp., was used for amoxicillin; and 500 mg of Klaricid^{®} film coated tablet of Abbott Korea Co., Ltd., was used for clarithromycin.

### Comparative Example 2: Lansoprazole, amoxicillin, and clarithromycin

30 mg of Lanston^{®} Capsule of Jeil Pharmaceutical Co., Ltd., was used for lansoprazole, 1000 mg of Kymoxin^{®} Capsule of Yuhan Corp., was used for amoxicillin, and 500 mg of Klaricid^{®} film coated tablet of Abbott Korea Co., Ltd., was used for clarithromycin.

### Example 3: Clinical study 1

### 1. Selection of subjects

### (1) Cohort 1

To evaluate pharmacokinetic interaction of multiple-dose administration of single and combination of tegoprazan and amoxicillin/clarithromycin, a clinical study was designed in accordance with a randomized, open-label, and multiple-dose design.

A total of 24 subjects who had been determined eligible to participate in the study were randomly assigned in a 1:1 ratio to each one of the two groups. Each randomly-assigned group was administered with investigational medical products as arranged during each intervention period and underwent a clinical study as designed. In consideration of the half-life of each product, a 14-day wash-out period was given between the intervention periods.

### (2) Cohort 2

To identify an effect of eradicating *Helicobacter pylori* in the present invention, a clinical study was designed in accordance with a randomized, open-label, active-controlled, parallel and multiple-dose design. A total of 221 patients were subjected to screening for this study. Out of them, 36 patients were registered thereto and the completed clinical results were obtained from 34 patients.

Particularly, the 36 patients above were divided into three patient groups (T1, T2 and R) and the clinical study was performed accordingly.

**[Table 1]**

| | |
|---|---|
| **T1** | CJ-12420 50 mg + Amoxicillin 1000 mg/Clarithromycin 500 mg |
| **(N=12)** | - Repeated administration twice a day for seven days |
| **T2** | CJ-12420 100 mg + Amoxicillin 1000 mg/Clarithromycin 500 mg |
| **(N=12)** | - Repeated administration twice a day for seven days |
| **R** | Pantoprazole 40 mg + Amoxicillin 1000 mg/Clarithromycin 500 mg |
| **(N=12)** | - Repeated administration twice a day for seven days |

### Inclusion criteria

Trial subjects should satisfy all the following selection criteria in order to participate in this clinical study, unless otherwise specified:
1) Healthy adults aged between 19 and 45 inclusively at the time of screening test;
2) No congenital or chronic disease, and no morbid symptoms or findings on examination;
3) Body mass index (BMI) of between 18.5 and 28.0 kg/m² inclusively;
4) Considered eligible based on medical examinations (including interview, vital signs, 12-lead ECG, physical examination, laboratory tests, etc.) which are set and performed by an investigator in accordance with profiles of an investigational product;
5) Voluntary consent to participate in the whole clinical study process after being fully informed of purpose and content of the study, profiles of the investigational product, etc., prior to the participation in this clinical study; and
6) Positive on ¹³C urea breath test.

### Exclusion criteria

The trial subjects were excluded from this clinical study, in the event that any of the followings occurs:
**1) Medical history**
   a) History or current evidence for diseases considered clinically relevant by the investigator, including hepatic, renal, gastrointestinal, respiratory, musculoskeletal, endocrine, neuropsychiatric, hemato-oncological, urinary, or cardiovascular (including cardiac arrhythmia) diseases;
   b) History of gastrointestinal diseases (e.g., gastritis, gastrospasm, gastroesophageal reflux disease, Crohn's disease, ulcer, etc.) or abdominal surgery (except for simple appendectomy or herniotomy) which are considered to have a potential effect on drug absorption by the investigator; and
   c) Previous treatment failure for *H. pylori* eradication.
**2) Laboratory tests and ECG**
   a) AST or ALT value is at least 1.25 times more than an upper limit of normal (ULN);
   b) Total bilirubin value is 1.5 times more than the ULN;
   c) eGFR calculated by CKD-EPI formula is less than 80 mL/min; and
   d) Any clinically relevant abnormalities on ECG.
**3) Allergy and drug abuse**
   a) History of hypersensitivity to this investigational product, components contained in the investigational product (penicillin, cephem and macrolide antibiotics, pantoprazole and benzimidazoles) and other drugs (including aspirin, antibiotics, etc.); and
   b) History of drug abuse or positive on drug screening test.
**4) Drug/dietary restrictions**
   a) Drugs (including herbal supplements) or abnormal diets (e.g., taking in at least 1 L of grapefruit juice, excessive garlic, broccoli, kale, etc.), which may have an effect on absorption, distribution, metabolism and excretion of the investigational product, within 28 days prior to the first study dose of the investigational product;
   b) Use of prescription drugs (ETCs), any over-the-counter drugs (OTCs), vitamins or the like within 10 days prior to the first study dose of the investigational product; and
   c) Participating in other clinical studies to receive other investigational products within three months prior to the first study dose of the investigational product (except for not taking in the investigational products).
**5) Blood donation and transfusion**
   a) Whole blood donation within 60 days prior to the first study dose of the investigational product; and
   b) Donation and transfusion of blood components within 30 days prior to the first study dose of the investigational product.
**6) Pregnancy and contraception**
   a) Pregnant, positive on pregnancy test, or breast-feeding; and
   b) Subject's or his spouse or partner's inability to use medically qualifying dual-contraceptive methods or medically acceptable contraceptive methods (including intrauterine devices with established pregnancy failure rates, concurrent use of physical barrier contraceptive method and spermicide, vasectomy, tubectomy/tubal ligation, hysterectomy, etc.) from screening to 30 days of the last dose of the investigational product.
**7) Others**
   a) Heavy use of alcohol in an amount of more than 30 g/day on average or positive on alcohol test;
   b) Heavy smoker with more than 10 cigarettes/day on average; and
   c) Caffeine intake in an amount of more than 400 mg/day; and
   d) Any clinically relevant findings considered inappropriate for clinical study participation at the discretion of the investigator.

### 2. Clinical study method

### (1) Cohort 1

A total of 24 subjects were divided into group 1 and group 2 . During the first intervention period, subjects of group 1 (12 person) were repeatedly administered with 100 mg of tegoprazan twice a day for four days and administered once a day on the fifth day on which a pharmacokinetic clinical study was conducted. During the second intervention period after a 14-day wash-out period, they were repeatedly administered with 1000 mg of amoxicillin and 500 mg of clarithromycin twice a day for four days and administered once a day on the fifth day. During the third intervention period after a 14-day wash-out period, they were repeatedly administered with 100 mg of tegoprazan, 1000 mg of amoxicillin, and 500 mg of clarithromycin twice a day for six days and administered once a day on the seventh day. Following the last dose of each intervention period, pharmacokinetic blood samples were collected for 17 times over 72 hours in the first period, 14 times over 48 hours in the second period, and 19 times over 120 hours in the third period.

Drugs were administered to subjects of group 2 (12 person) in the reverse order to the way the study was conducted on group 1 in the first and second intervention periods and in the same order in the third intervention period. Pharmacokinetic blood samples were collected in the same manner as group 1.

### (2) Cohort 2

As a result of screening (Day 28 - Day 2) before administering the first dose of the investigative product, a total of 36 subjects, who had satisfied the selection criteria and the exclusion criteria (T1-12 subjects, T2-12 subjects and R-12 subjects), participated in a study to evaluate a change of an intragastric pH and an eradication rate upon a repeated co-administration of 50 mg/100 mg of CJ-12420 and amoxicillin/clarithromycin, and upon a repeated co-administration of 40 mg of pantoprazole and amoxicillin/clarithromycin.

The T1 group was repeatedly given 50 mg of CJ-12420 and 1000 mg of amoxicillin/500 mg of clarithromycin twice a day for seven days; the T2 group was repeatedly given 100 mg of CJ-12420 and 1000 mg of amoxicillin/500 mg of clarithromycin twice a day for seven days; and the R group was repeatedly given 40 mg of pantoprazole and 1000 mg of amoxicillin/500 mg of clarithromycin twice a day for seven days. An intragastric pH was measured from all the subjects on Day 1 for 24 hours, and the intragastric pH was also measured for 24 hours on Day 1 and Day 7 of repeated administration. Also, a negative conversion rate was calculated upon a follow-up visit paid by subjects who were identified to be *H. pylori* positive on the UBT test during screening, and stability evaluation was performed according to a pre-determined schedule.

### 3. Evaluation item

### (1) Pharmacodynamic evaluation

### Evaluation variables

Evaluation of median pH, percentage of time with pH > 6 and rate of success in eradication

### (2) Pharmacokinetic evaluation

### Evaluation parameters

The average plasma concentrations of tegoprazan, clarithromycin, and amoxicillin were measured and time-based plasma concentration and time-based drug concentration patterns were observed when tegoprazan was repeatedly administered alone, amoxicillin/clarithromycin were repeatedly administered and tegoprazan, amoxicillin, and clarithromycin were repeatedly administered in combination.

### 4. Statistical analysis

### (1) Demographic information

Descriptive statistics were presented with regard to key demographic variables (age, weight, height, etc.).

### (2) Pharmacodynamic evaluation index

Descriptive statistics were presented with regard to an intragastric pH for each administration group.

Also, a rate of success in eradication was calculated with regard to a negative conversion rate calculated as a result of UBT.

### (3) Pharmacokinetic evaluation index

Pharmacokinetics of tegoprazan, amoxicillin, and clarithromycin were evaluated. Descriptive statistics of pharmacokinetic evaluation parameters were presented. Point estimates for geometric mean ratio between each group and 90% confidence interval were calculated from the log-converted primary pharmacokinetic evaluation parameters by using a linear mixed effect model.

### 5. Results

### (1) Subject participation status and demographic distribution

### 1) Cohort 1

Among 24 subjects registered in Cohort 1, 20 subjects completed the entire clinical study; among four dropouts, three withdrew their consent and one was dropped out according to the principal investigator's decision. All subjects were male and their average age, height, and weight were 27.3 ± 4.41 years old, 172.92 ± 5.696 cm, and 70.04 ± 8.396 kg.

### 2) Cohort 2

Out of 221 volunteers who had received a screening test, 36 volunteers (T1: 12, T2: 12 and R: 12) were registered to the clinical study, out of which 34 subjects completed all the clinical study processes, except for two drop-outs (T2: 1 and R: 1). The subjects were all male, wherein their average age, height and weight were 25.6±3.26 years old, 175.98±4.902 cm and 74.28±8.721 kg, respectively.

### (2) Pharmacodynamic evaluation

On each of Day 1 and Day 7, a median pH value and a percentage of time at which an intragastric pH is maintained at 6 or more were summarized in Fig. 1 or Fig. 2, respectively.

Fig. 1 shows a change of a mean median pH with time and a percentage of time at which the intragastric pH is maintained at 6 or more on Day 1, and Fig. 2 shows a change of a mean median pH with time and a percentage of time at which the intragastric pH is maintained at 6 or more on Day 7.

The mean median pH of baseline (Day 1) was 2.2±1.03, 2.38±1.17 and 1.87±0.44 in T1, T2 and R administration groups, respectively, and the mean median pH of Day 1 was 7.23±0.47, 7.5±0.31 and 5.11±2.18, such that a remarkable increase was observed compared to the baseline.

The mean median pH of Day 7 was 6.94±0.45, 7.33±0.56 and 6.01±1.44 in T1, T2 and R administration groups, respectively, and thus it was identified that the mean median pH of Day 7 is similar to the mean median pH of Day 1 in T1 and T2 administration groups; and the mean median pH of Day 7 is increased more than that of Day 1 in R administration group, but still lower compared to T1 or T2 group.

Also, a percentage of time at which the intragastric pH is maintained at 6 or more during 24 hours of the baseline (Day 1) was 10.19±13.16% in T1 administration group, 15.33+10.61% in T2 administration group, and 8.18±6.96% in R administration group, respectively. On Day 1, the percentage of time was 87.7±10.71% in T1 administration group, 96.26±1.49% in T2 administration group, and 49.71±29.49% in R administration group, such that a remarkable increase was observed compared to the baseline.

The percentage of time at which the intragastric pH is maintained at 6 or more on Day 7 was 88.03±8.73% in T1 administration group, 96.33±5.55% in T2 administration group and 58.34±29.21% in R administration group, respectively, and thus an increase on Day 7 was observed compared to Day 1, but without a remarkable difference therebetween, and a considerably low value was shown compared to T1 or T2 administration group dosed with Tegoprazan.

As identified above, the administration of Tegoprazan in the present invention greatly increased the mean median pH as well as the time at which the intragastric pH was maintained at 6 or more during 24 hours. Also, such administration greatly increased pH to 6 or more within a short time of two hours or less right after the administration.

On the other hand, a case of using pantoprazole was characterized in that a speed of pH increase is slow right after the administration thereof, and the median pH and the time at which the intragastric pH is maintained at 6 or more during 24 hours are also less compared to the present invention.

In other words, it was shown that using of Tegoprazan of the present invention maintains pH close to a pKa value of antibiotics, i.e., amoxicillin and clarithromycin to enhance stability of the antibiotics and greatly reduce a minimum inhibitory concentration of the antibiotics, and also has a long half life to maintain a rising state of an intragastric pH for a certain period of time or longer, such that using of Tegoprazan may maximize an eradication effect of amoxicillin and clarithromycin.

### (3) Pharmacokinetic parameter evaluation

Pharmacokinetic characteristics of tegoprazan were evaluated after repeatedly administering 100 mg of tegoprazan twice a day alone, repeatedly administering 1000 mg of amoxicillin and 500 mg of clarithromycin twice a day in combination, or repeatedly administering 100 mg of tegoprazan, 1000 mg of amoxicillin, and 500 mg of clarithromycin twice a day in combination. As a result, Cmax of tegoprazan increased by 2.24 times and AUC increased by 2.70 times when administered in combination with amoxicillin/clarithromycin; Cmax and AUC of clarithromycin showed a tendency to increase when administered in combination with tegoprazan. It was, therefore, confirmed that in the case of administering these three formulations in combination, the bioavailability of tegoprazan and clarithromycin will increase.

### Example 4: Clinical study 2

### 1. Selection of subjects

To identify an effect of eradicating *Helicobacter pylori* in the present invention, a clinical study was designed in accordance with a randomized, open-label, active-controlled, parallel and multiple-dose design. A total of 350 patients screened and the 350 patients were registered to conduct clinical trials.

### Inclusion Criteria

Unless otherwise specified, participants of the study were required to meet all the inclusion criteria below.
(1) Healthy adults aged between 20 and 75 at the time of screening
(2) Those who complain of having epigastric discomfort
(3) *H. pylori* positive as a result of screening
(4) Those who fall under any one of the following cases on upper gastrointestinal endoscopy as a result of screening:
   - Peptic ulcer (gastric ulcer or duodenal ulcer)
   - Diagnosis of scarred gastric ulcer or duodenal ulcer
   - History of gastric ulcer or duodenal ulcer on medical record, though there is no scar
   - Chronic atrophic gastritis
(5) Those who can understand and comply with instructions and participate in the entire study
(6) Those who voluntarily decide to participate in the study and sign a written consent form
(7) Those who agree to the use of medically valid contraceptive measures (including medically infertile state) during the study
   - Female volunteers who are medically infertile can participate in the study:
      Those who underwent menopause (amenorrhoea for 24 or more months), hysterectomy, salpingectomy, or bilateral ovariectomy
   - Medically valid contraceptive measures: device in the uterus (loop, Mirena), physical contraception (male condom, female condom (femidom)), subdermal contraceptive implant (Implanon), time-release contraceptive injections or tubectomy and tubal ligation, and vasectomy (however, oral contraceptives are not allowed during the study and dual contraception is recommended during the study to prevent pregnancy)

### Exclusion Criteria

Participants were excluded from the study if they met any one of the following criteria.
(1) Unable to undergo upper gastrointestinal endoscopy
(2) Those who received *H. pylori* eradication therapy
(3) Those who have acute upper gastrointestinal bleeding, acute gastric mucosal lesion (AGML), or acute duodenal mucosal lesion (ADML) on upper gastrointestinal endoscopy
(4) Those who underwent or will undergo surgery that could affect the secretion of gastric acid (e.g. upper gastrointestinal resection or vagotomy)
   - However, simple perforation surgery, appendectomy, cholecystectomy, and endoscopic removal of benign tumors are excluded
(5) History of Zollinger-Ellison syndrome or gastric acid hypersecretion disorders
(6) Those who were diagnosed with gastric outlet obstruction (GOO) or are diagnosed with gastric cancer on upper gastrointestinal endoscopy at screening
(7) Those who were given proton pump inhibitors (PPI) or histamine H2-receptor antagonists at full dose within 14 days before screening
(8) Those who were given antibiotics for *H. pylori* eradication or drugs containing bismuth formulations within 28 days before screening
(9) Pregnant woman or breastfeeding woman
(10) Clinically meaningful abnormal results at screening
   - AST, ALT, ALP, γ-GT or total bilirubin values are two or more times higher than upper normal limit (UNL) of each testing institution
   - BUN or creatinine values are 1.5 or more times higher than upper normal limit (UNL) of each testing institution
(11) Clinically meaningful abnormal electrocardiogram
   - Major arrhythmia, multifocal PVC, or 2°AV-block abnormality
(12) History of malignant tumors in five years
   - However, if a participant had attained complete remission (CR, pCR) of tumors and had no recurrence in over five years or a participant, after completely removing tumors by endoscopic resection, had no recurrence in over three years, it is allowed to be included.
(13) Clinically meaningful abnormalities in the liver, kidney, cardiovascular system, respiratory system, endocrine system, or central nervous system
(14) History of hypersensitivity reactions to the main ingredient or bulking agent of tegoprazan or proton pump inhibitors (PPI), penicillin antibiotics, and macrolides antibiotics
(15) Those who are scheduled to have surgery requiring in-hospital care or require surgical treatment during the study
(16) Those who participated in other clinical studies (except for CJ_APA_303 clinical study) within four weeks based on Second Visit (randomized clinical study date)
   - Those who participated in or are participating in non-interventional study (observational study or survey) that would not impact the outcome and safety evaluation of the current study, as determined by the investigator, may participate in the study. In addition, those who signed a written consent form to participate in other clinical study but were eliminated after screening without any administration of investigational products or care may participate in the study.
   - However, if a participant participated in [CJ_APA_303] study and it was confirmed that the ulcer had been cured, it is allowed to participate in the study.
(17) In addition to the above criteria, those who are not eligible for the study according to the investigator's clinically meaningful opinion based on medical determination

### 2. Clinical study method

A total of 350 subjects (TAC -175, LAC-175) who met all inclusion criteria and no exclusion criteria as a result of screening (endoscopy) on First Visit before the administration of the first dose of investigational products (D-14 - D-0) participated in the study to evaluate *H. pylori* eradication rates when tegoprazan 50 mg and amoxicillin/clarithromycin are repeatedly administered in combination and lansoprazole 30 mg and amoxicillin/clarithromycin are repeatedly administered in combination.

TAC group was repeatedly administered with tegoprazan 50 mg, amoxicillin 1000 mg/clarithromycin 500 mg twice a day for seven days, and LAC group was repeatedly administered with lansoprazole 30 mg, amoxicillin 1000 mg/clarithromycin 500 mg twice a day for seven days.

Following the completion of eligibility evaluation through screening, it was advised to take the investigational products prescribed on Second Visit (Day 0) starting the next day morning (Day 1). A drug administration log of the participant of the study was recorded from the first day of starting the administration of investigational products.

Participants were advised to arrive on empty stomach without taking any investigational products on Third Visit (Day 8).

### 3. Evaluation item

(1) *H. pylori* eradication rate according to MIC of clarithromycin
   MIC criteria for clarithromycin is described below.
   ≤0.5 (*µ*g/mℓ): Susceptible and Intermediate
   ≥1 (*µ*g/mℓ): Resistant
*(2) H. pylori* eradication rate according to lesions on upper gastrointestinal endoscopy

### 4. Results

Based on *H. pylori* eradication rates according to clarithromycin MIC, it was found that TAC group, wherein tegoprazan 50 mg and amoxicillin/clarithromycin had been repeatedly administered in combination, had an *H. pylori* eradication success rate of 92% and was more excellent in *H. pylori* eradication than LAC group, wherein lansoprozol 30 mg and amoxicillin/clarithromycin had been repeatedly administered in combination. The *H. pylori* eradication effect was also observed in patients with bacterial resistance when tegoprazan 50 mg and amoxicillin/clarithromycin were repeatedly administered in combination.

In addition, based on *H. pylori* eradication rates according to lesions on upper gastrointestinal endoscopy, TAC group had an *H. pylori* eradication rate of 76.19% in pepti ulcer disease (PUD) and was more excellent in *H. pylori* eradication than LAC group. TAC group also had an *H. pylori* eradication rate of 68.15% in chronic atrophic gastritis (CAG) and had an eradication effect that was equivalent to or stronger than that of LAC group.

The above results demonstrate that the pharmaceutical composition for use in eradicating *Helicobacter pylori* according to the present invention could have an excellent effect of eradicating *Helicobacter pylori* by maximizing eradication effects of amoxicillin and clarithromycin.

### [Industrial Applicability]

A pharmaceutical composition for use in eradicating *Helicobacter pylori* according to the present invention maintains an intragastric pH at a certain level or more for a certain period of time or longer, thereby maintaining pH close to a pKa value of amoxicillin and clarithromycin to enhance stability of antibiotics, and thereby greatly reducing a minimum inhibitory concentration of the antibiotics to maximize an eradication effect of amoxicillin and clarithromycin. Also, such composition shows an excellent effect on eradicating even *Helicobacter pylori* resistant to antibiotics, and thus may be also effectively used in eradicating resistant strains, and such composition continues to have an effect of maintaining pH at a certain level or more for a long time, and thus has an advantage of achieving a remarkably high compliance with medication, too. Furthermore, such composition has an advantage, in that it may be available before or after meals without considering a diet therapy, unlike existing combination therapies, and thus is expected to be valuably used in a related pharmaceutical industry field.

## Claims

1. A pharmaceutical composition for use in eradicating *Helicobacter pylori,* comprising: a compound represented by a following formula 1, optical isomers thereof or pharmaceutically acceptable salts thereof; amoxicillin or pharmaceutically acceptable salts thereof; and clarithromycin or pharmaceutically acceptable salts thereof as an active ingredient:

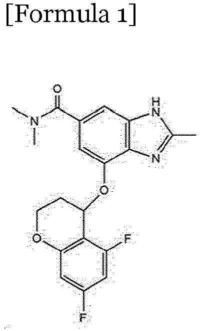

2. The pharmaceutical composition for use according to claim 1, wherein the compound represented by the formula 1 is a compound represented by a following formula 2:

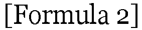

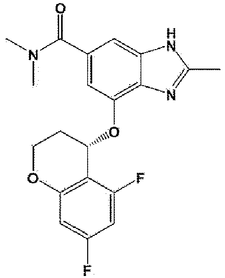

3. The pharmaceutical composition for use according to claim 1, wherein the composition comprises 10 mg to 500 mg of the compound represented by the formula 1 or pharmaceutically acceptable salts thereof, 100 mg to 4 g of amoxicillin or pharmaceutically acceptable salts thereof, and 50 mg to 3 g of clarithromycin or pharmaceutically acceptable salts thereof.

4. The pharmaceutical composition for use according to claim 1, wherein the pharmaceutical composition increases an intragastric pH to 5 or more within three hours after administration thereof.

5. The pharmaceutical composition for use according to claim 1, wherein the pharmaceutical composition eradicates *Helicobacter pylori* resistant to antibiotics, preferably wherein the *Helicobacter pylori* resistant to antibiotics is resistant to amoxicillin or clarithromycin.

6. The pharmaceutical composition for use according to claim 1, wherein the pharmaceutical composition is administered into subjects infected with *Helicobacter pylori.*

7. The pharmaceutical composition for use according to claim 6, wherein the *Helicobacter pylori* is *Helicobacter pylori* resistant to antibiotics.

8. The pharmaceutical composition for use according to claim 7, wherein the *Helicobacter pylori* resistant to antibiotics is resistant to amoxicillin or clarithromycin.

9. The pharmaceutical composition for use according to claim 1, wherein the pharmaceutical composition is an oral administration type.

10. A combination, comprising: a compound represented by a following formula 1, optical isomers thereof or pharmaceutically acceptable salts thereof; amoxicillin or pharmaceutically acceptable salts thereof; and clarithromycin or pharmaceutically acceptable salts thereof:

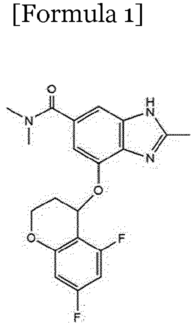

11. The combination according to claim 10, wherein the combination is for use in eradicating *Helicobacter pylori.*

12. A kit, comprising a combination for use in eradicating *Helicobacter pylori,* comprising: a compound represented by a following formula 1, optical isomers thereof or pharmaceutically acceptable salts thereof; amoxicillin or pharmaceutically acceptable salts thereof; and clarithromycin or pharmaceutically acceptable salts thereof:

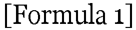

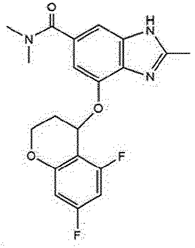

## Patentansprüche

1. Pharmazeutische Zusammensetzung zur Verwendung bei der Eradikation von *Helicobacter pylori,* umfassend: eine Verbindung, dargestellt durch die folgende Formel 1, optische Isomere davon oder pharmazeutisch akzeptable Salze davon; Amoxicillin oder pharmazeutisch akzeptable Salze davon und Clarithromycin oder pharmazeutisch akzeptable Salze davon als einen Wirkstoff:

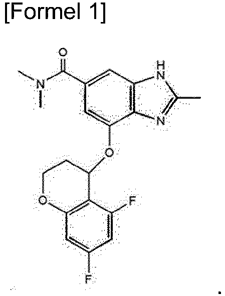

2. Pharmazeutische Zusammensetzung zur Verwendung nach Anspruch 1, wobei die durch die Formel 1 dargestellte Verbindung eine durch die Formel 2 dargestellte Verbindung ist:

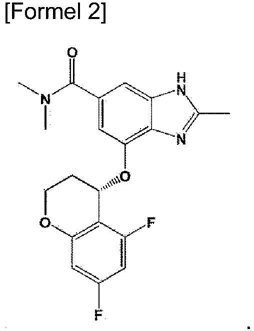

3. Pharmazeutische Zusammensetzung zur Verwendung nach Anspruch 1, wobei die Zusammensetzung 10 mg bis 500 mg der durch die Formel 1 dargestellten Verbindung oder pharmazeutisch akzeptabler Salze davon, 100 mg bis 4 g Amoxicillin oder pharmazeutisch akzeptabler Salze davon und 50 mg bis 3 g Clarithromycin oder pharmazeutisch akzeptabler Salze davon umfasst.

4. Pharmazeutische Zusammensetzung zur Verwendung nach Anspruch 1, wobei die pharmazeutische Zusammensetzung innerhalb von drei Stunden nach ihrer Verabreichung den intragastrischen pH auf 5 oder mehr erhöht.

5. Pharmazeutische Zusammensetzung zur Verwendung nach Anspruch 1, wobei die pharmazeutische Zusammensetzung gegen Antibiotika resistentes *Helicobacter pylori* ausrottet, wobei das gegen Antibiotika resistente *Helicobacter pylori* vorzugsweise gegen Amoxicillin oder Clarithromycin resistent ist.

6. Pharmazeutische Zusammensetzung zur Verwendung nach Anspruch 1, wobei die pharmazeutische Zusammensetzung mit *Helicobacter pylori* infizierten Individuen verabreicht wird.

7. Pharmazeutische Zusammensetzung zur Verwendung nach Anspruch 6, wobei das *Helicobacter pylori* gegen Antibiotika resistentes *Helicobacter pylori* ist.

8. Pharmazeutische Zusammensetzung zur Verwendung nach Anspruch 7, wobei das gegen Antibiotika resistente *Helicobacter pylori* gegen Amoxicillin oder Clarithromycin resistent ist.

9. Pharmazeutische Zusammensetzung zur Verwendung nach Anspruch 1, wobei die pharmazeutische Zusammensetzung zur oralen Verabreichung vorgesehen ist.

10. Kombination, umfassend: eine durch die folgende Formel 1 dargestellte Verbindung, optische Isomere davon oder pharmazeutisch akzeptable Salze davon; Amoxicillin oder pharmazeutisch akzeptable Salze davon und Clarithromycin oder pharmazeutisch akzeptable Salze davon:

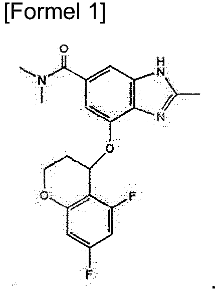

11. Kombination nach Anspruch 10, wobei die Kombination zur Verwendung bei der Eradikation von *Helicobacter pylori* vorgesehen ist.

12. Kit, umfassend eine Kombination zur Verwendung bei der Eradikation von *Helicobacter pylori,* umfassend: eine Verbindung, dargestellt durch die folgende Formel 1, optische Isomere davon oder pharmazeutisch akzeptable Salze davon; Amoxicillin oder pharmazeutisch akzeptable Salze davon und Clarithromycin oder pharmazeutisch akzeptable Salze davon:

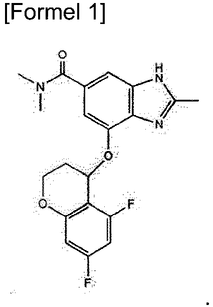

## Revendications

1. Composition pharmaceutique utilisée pour éradiquer l'*Helicobacter pylori,* comprenant : un composé représenté par la formule 1 suivante, ses isomères optiques ou ses sels pharmaceutiquement acceptables ; l'amoxicilline ou ses sels pharmaceutiquement acceptables ; et la clarithromycine ou ses sels pharmaceutiquement acceptables en tant qu'ingrédient actif :

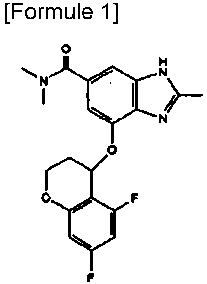

2. Composition pharmaceutique à utiliser selon la revendication 1, dans laquelle le composé représenté par la formule 1 est un composé représenté par la formule 2 suivante :

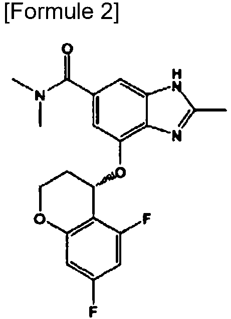

3. Composition pharmaceutique selon la revendication 1, dans laquelle la composition comprend 10 mg à 500 mg du composé représenté par la formule 1 ou de ses sels pharmaceutiquement acceptables, 100 mg à 4 g d'amoxicilline ou de ses sels pharmaceutiquement acceptables, et 50 mg à 3 g de clarithromycine ou de ses sels pharmaceutiquement acceptables.

4. Composition pharmaceutique selon la revendication 1, dans laquelle la composition pharmaceutique augmente le pH intragastrique à 5 ou plus dans les trois heures suivant son administration.

5. Composition pharmaceutique selon la revendication 1, dans laquelle la composition pharmaceutique éradique l'*Helicobacter pylori* résistant aux antibiotiques, de préférence dans laquelle l'*Helicobacter pylori* résistant aux antibiotiques est résistant à l'amoxicilline ou à la clarithromycine.

6. Composition pharmaceutique utilisée selon la revendication 1, dans laquelle la composition pharmaceutique est administrée à des sujets infectés par l'*Helicobacter pylori.*

7. Composition pharmaceutique selon la revendication 6, dans laquelle l'*Helicobacter pylori* est l'*Helicobacter pylori* résistant aux antibiotiques.

8. Composition pharmaceutique selon la revendication 7, dans laquelle l'*Helicobacter pylori* résistant aux antibiotiques est résistant à l'amoxicilline ou à la clarithromycine.

9. Composition pharmaceutique utilisée selon la revendication 1, dans laquelle la composition pharmaceutique est un type d'administration orale.

10. Combinaison comprenant : un composé représenté par la formule 1 suivante, ses isomères optiques ou ses sels pharmaceutiquement acceptables ; l'amoxicilline ou ses sels pharmaceutiquement acceptables ; et la clarithromycine ou ses sels pharmaceutiquement acceptables :

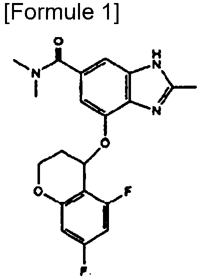

11. Combinaison selon la revendication 10, dans laquelle la combinaison est utilisée pour éradiquer l'*Helicobacter pylori.*

12. Kit comprenant une combinaison destinée à éradiquer l'*Helicobacter pylori,* comprenant : un composé représenté par la formule 1 suivante, ses isomères optiques ou ses sels pharmaceutiquement acceptables ; de l'amoxicilline ou ses sels pharmaceutiquement acceptables ; et de la clarithromycine ou ses sels pharmaceutiquement acceptables :
